# EUROPEAN PATENT APPLICATION

(11) **EP 4 183 344 A1**
(43) Date of publication of application: **24.05.2023**
(21) Application number: 21842070.1
(22) Date of filing: 28.05.2021
(51) Int. Cl.: A61B 6/00, A61B 6/03, G16H 50/20, G16H 30/40, G06T 7/00

(54) **DEVICE AND METHOD FOR SUPPORTING CHEST MEDICAL IMAGE READING**

(30) Priority: 16.07.2020 KR 20200088088
(71) Applicant: Coreline Soft Co., Ltd, Seoul 03991 (KR); Institute for Diagnostic Accuracy Research BV, 9713 GH Groningen (NL)
(72) Inventor: YI, Jaeyoun, Seoul 02831 (KR); YU, Donghoon, Gimpo-si, Gyeonggi-do 10073 (KR); KWON, Sunggoo, Guri-si, Gyeonggi-do 11902 (KR); OUDKERK, Matthijis, 8355 BM Giethoorn (NL)
(74) Representative: Stöckeler, Ferdinand
(86) International application number: PCT/KR2021/006670
(87) International publication number: WO 2022/014856

(57) **Abstract**

Disclosed therein are a medical image reading assistance apparatus and method for assisting the reading of chest medical images. The medical image reading assistance apparatus includes a computing system, and the computing system includes at least one processor. The at least one processor segments airways and blood vessels from a medical image including the lungs, segments small regions corresponding to a plurality of intensity sections divided and set based on at least one of the property and state of tissue in a lung area image generated by excluding the airways and the blood vessels from the medical image, and visualizes the distributions of the small regions corresponding to the plurality of intensity sections within the lung area image.

## Description

### TECHNICAL FIELD

The present invention relates to an apparatus and method for assisting the reading of medical images. More particularly, the present invention relates to an apparatus (a computing system) for visualizing the results of the analysis of medical images to assist medical staff in effectively reading chest medical images, and also relates to software that is executed in the apparatus.

### BACKGROUND ART

Currently, medical images such as computed tomography (CT) images are widely used to analyze lesions and use analysis results for diagnosis. For example, chest CT images are frequently used for reading because they enable readers to observe abnormalities in parts of the human body, such as the lungs, the bronchi, and the heart.

Some of the findings that can be read through chest CT images may be easily overlooked by human doctors because they are not easy to read and even radiologists can distinguish their features and forms only after years of training. In particular, when the level of difficulty in reading is high as in the reading of a lung nodule, the case of overlooking a lesion may occur even when a doctor pays a high degree of attention, which may lead to trouble.

In order to assist in reading images that humans can easily overlook, the need for computer-aided diagnosis (CAD) has arisen. Conventional CAD technology is limited to assisting doctors in making decisions in a significantly limited area.

For example, Korean Patent Application Publication No. 10-2019-0090986 entitled "System and Method for Assisting Reading of Chest Medical Images" discloses a technology that is configured to segment a lung area based on the distribution of intensity values in a chest medical image, especially a chest CT image, and detect a lesion within the segmented lung area.

In general, the intensity of a CT image varies depending on the characteristics of a human organ and tissue in a CT image. Accordingly, a technology for identifying a normal tissue and a lesion by segmenting regions having similar intensity values is obvious to those skilled in the art.

In the field of medical imaging diagnosis, the task of detecting ground-glass opacity (GGO) in a lung area is significantly important. GGO refers to local nodular pulmonary infiltration, and is generally defined as a nodule in which the boundaries of bronchi or blood vessels are drawn desirably. Korean Patent Application Publication No. 10-2019-0090986 discloses the concept of adaptively applying an intensity threshold so that GGO can be found well using artificial intelligence.

As another prior art document, Korean Patent Application Publication No. 10-2020-0046507 entitled "Method of Providing Information for Diagnosis of Pulmonary Fibrosis and Computing Apparatus for Diagnosing Pulmonary Fibrosis" discloses a technology that is configured to analyze the spatial distribution of lesion tissue by searching for the terminal points of normal tissues of the lung of a patient in a medical image in order to diagnose the fibrosis of lung tissue. In other words, Korean Patent Application Publication No. KR 10-2020-0046507 discloses a technology for assisting the diagnosis of pulmonary fibrosis based on the intensity of a medical image and by taking into consideration spatial distribution.

As still another prior art document, Korean Patent No. 10-1587719 entitled "Medical Image Analysis Apparatus and Method for Distinguishing between Pulmonary Nodule and Pulmonary Vessel" discloses a technology that aims to detect GGO, which is rounded opacity whose boundaries are drawn desirably in a chest medical image and is configured to distinguish between a pulmonary nodule and a pulmonary blood vessel by taking into consideration the mobility of a detected object as well as to simply detect GGO using only intensity.

However, the conventional technologies disclosed in these prior art documents are configured to detect GGO candidates using CT intensity values in a medical image including a lung area and then diagnose a disease by taking into consideration an index such as the spatial distribution of the GGO candidates or the mobility of an object. As a result, these conventional technologies provide only information about symptoms of lung disease, but it is difficult for the conventional technologies to provide additional information about causes of the lung disease.

### DISCLOSURE OF INVENTION

### PROBLEM TO BE SOLVED

Recently, it is known that lung diseases can be caused by various causes, as in the case of viral lung diseases such as COVID-19 and bacterial lung diseases. Accordingly, there is an increasing demand for technology for assisting the reading of chest medical images that can not only detect a symptom of lung disease but can also provide additional information about a cause of the lung disease.

The conventional technologies disclosed in the prior art documents are configured to diagnose a lung disease by detecting GGO candidates using CT intensity values in a medical image including a lung area and then taking into consideration an index such as the spatial distribution of the GGO candidates or the mobility of an object. As a result, the conventional technologies provide only information about a symptom of the lung disease, and it is difficult for the conventional technologies to provide additional information about a cause of the lung disease.

An object of the present invention is to detect various factors inside a lung area and provide medical staff with information about the factors together with information about a symptom of lung disease. In other words, an object of the present invention is to set various intensity sections based on the state of tissue inside a lung area and/or the nature of the tissue and visualize the intensity sections, thereby assisting medical staff in identifying symptoms of lung disease and then obtaining additional information about various factors that are causes of the symptoms.

For example, a lung disease caused by COVID-19 may have a different pattern from the pattern of a general bacterial lung disease. The present invention is configured to identify additional information about this pattern inside a lung area, visualize the additional information, and provide medical staff with the additional information together with information about a symptom of lung disease, thereby assisting the reading of a chest medical image so that the medical staff can take into consideration various factors in connection with causes of the lung disease.

Due to the recent epidemic of COVID-19, it has been recognized by medical staff that if the causes of lung diseases are different even when the symptoms of the lung diseases are similar, treatment methods and the types of drugs to be administered need to be completely different. However, since the conventional medical image reading assistance technology provides only information about symptoms of lung disease, it has been difficult to assist medical staff in diagnosis and decision making. An object of the present invention is to provide technology for assisting the reading of chest medical images that provides information about symptoms of lung disease and additional information for the estimation of the causes of the symptoms together, thereby assisting medical staff in reading, diagnosis, and decision making.

An object of the present invention is to provide technology for assisting the reading of chest medical images that visualizes additional information for distinguishing between a lung disease caused by COVID-19 and a general bacterial lung disease, thereby assisting medical staff in rapid and accurate decision making.

An object of the present invention is to implement a platform that provides a user menu to allow a user to reset or add one or more intensity sections of clinical tissue based on updated clinical knowledge when the clinical knowledge on a cause of lung disease is updated and also provides the user with reading assistance information about various causes of lung disease together with information about symptoms of the lung disease.

An object of the present invention is to quantify the distribution of specific intensity regions for a specific cause of lung disease, generate reading assistance information about whether the lung disease is present and the specific cause of the lung disease based on the distribution of the specific intensity regions, and then provide the reading assistance information to a user.

### TECHNICAL SOLUTION

According to an aspect of the present invention, there is provided a medical image reading assistance apparatus for assisting the reading of chest medical images, the medical image reading assistance apparatus including a computing system, wherein the computing system includes at least one processor. In this case, the at least one processor is configured to: segment airways and blood vessels from a medical image including the lungs; segment small regions corresponding to a plurality of intensity sections divided and set based on at least one of the property and state of tissue in a lung area image generated by excluding the airways and the blood vessels from the medical image; and visualize the distributions of the small regions corresponding to the plurality of intensity sections within the lung area image.

The plurality of intensity sections may be set based on at least one of the clinically distinctive property and state of tissue.

The at least one processor may be further configured to threshold the small regions based on the plurality of intensity sections within the lung area image.

The at least one processor may be further configured to, within the lung area image, visualize the distribution of first small regions corresponding to a first intensity section on a first window and also visualize the distribution of second small regions corresponding to a second intensity section on a second window.

The at least one processor may be further configured to, within the lung area image, visualize the distribution of first small regions corresponding to a first intensity section and also visualize the distribution of second small regions corresponding to a second intensity section by overlaying the distribution of second small regions on the distribution of first small regions.

The at least one processor may be further configured to provide a user menu configured to allow a user to reset the plurality of intensity sections or add one or more intensity sections to the plurality of intensity sections.

According to another aspect of the present invention, there is provided a medical image reading assistance apparatus for assisting the reading of chest medical images, the medical image reading assistance apparatus including a computing system, wherein the computing system includes at least one processor. In this case, the at least one processor is configured to: segment airways and blood vessels from a medical image including the lungs; segment first regions corresponding to a first intensity section set to include at least one of blood and thrombi in a lung area image generated by excluding the airways and the blood vessels from the medical image; and visualize the distribution of the first regions within the lung area image.

The first intensity section may be set to correspond to blood.

The first intensity section may be set to correspond to thrombi.

The at least one processor may be further configured to: segment second regions corresponding to the second intensity section set to correspond to blood within the lung area image; and visualize the distribution of the first regions and the distribution of the second regions so that the first regions and the second regions are distinguished from each other within the lung area image.

The at least one processor may be further configured to: segment third regions corresponding to a third intensity section set to correspond to ground-glass opacity (GGO) within the lung area image; and visualize the distribution of the first regions and the distribution of the third regions so that the first regions and the third regions are distinguished from each other within the lung area image.

The at least one processor may be further configured to threshold the first regions based on the first intensity section within the lung area image.

The at least one processor may be further configured to: quantify the distribution of the first regions within the lung area image; and generate reading assistance information regarding whether a lung disease is present in the lung area image and a cause of the lung disease based on information about the quantification of the distribution of the first regions.

According to still another aspect of the present invention, there is provided a medical image reading assistance method, the medical image reading assistance method being performed by a medical image reading assistance apparatus for assisting the reading of chest medical images, the medical image reading assistance apparatus including a computing system that includes at least one processor, the medical image reading assistance method being performed by program instructions that are executed by the at least one processor.

The medical image reading assistance method includes: segmenting airways and blood vessels in a medical image including the lungs; generating a lung area image by excluding the airways and the blood vessels from the medical image; segmenting small regions corresponding to a plurality of intensity sections divided and set based on at least one of the property and state of tissue in the lung area image; and visualizing the distributions of the small regions corresponding to the plurality of intensity sections within the lung area image.

According to still another aspect of the present invention, there is provided a medical image reading assistance method, the medical image reading assistance method including: segmenting airways and blood vessels in a medical image including the lungs; generating a lung area image by excluding the airways and the blood vessels from the medical image; segmenting first regions corresponding to a first intensity section set to include at least one of blood and thrombi in the lung area image; and visualizing the distribution of the first regions within the lung area image.

### ADVANTAGOUS EFFECTS

According to the present invention, there may be implemented technology for assisting the reading of chest medical images that can not only detect a symptom of lung disease, but also provide additional information about a cause of the lung disease.

According to the present invention, various factors inside a lung area may be detected and medical staff may be provided with information about the factors together with information about a symptom of lung disease. Various intensity sections may be set based on the state of tissue inside a lung area and/or the nature of the tissue and then visualized, thereby assisting medical staff in identifying a symptom of lung disease and then obtaining additional information about various factors.

According to the present invention, additional information for distinguishing between a lung disease caused by COVID-19 and a general bacterial lung disease may be visualized, thereby assisting medical staff in rapid and accurate decision making.

According to the present invention, a user menu may be provided to allow a user to reset or add one or more intensity sections of clinical tissue based on updated clinical knowledge when the clinical knowledge on a cause of lung disease is updated. There may be implemented a platform that provides a user with reading assistance information about various causes of lung disease together with information about symptoms of the lung disease.

According to the present invention, the distribution of specific intensity regions for a specific cause of lung disease may be quantified. Furthermore, reading assistance information about whether the lung disease is present and the specific cause of the lung disease may be generated based on the distribution of the specific intensity regions and then provided to a user.

### DESCRIPTION OF THE DRAWINGS

The above and other objects, features and advantages of the present invention will be more clearly understood from the following detailed description taken in conjunction with the accompanying drawings, in which:
FIG. 1 is a drawing showing a medical image reading assistance apparatus for assisting the reading of chest medical images according to an embodiment of the present invention;
FIG. 2 is a diagram showing the sub-modules of a program instruction set executed in a medical image reading assistance apparatus according to an embodiment of the present invention and also showing the main process of a medical image reading assistance method performed according to the execution of the sub-modules;
FIG. 3 is a diagram showing the sub-modules of a program instruction set executed in a medical image reading assistance apparatus according to an embodiment of the present invention and also showing the main process of a medical image reading assistance method performed according to the execution of the sub-modules;
FIG. 4 is a diagram illustrating an embodiment of a plurality of intensity sections based on a plurality of properties and states of clinical tissues applied in a medical image reading assistance apparatus according to an embodiment of the present invention;
FIG. 5 shows views illustrating an example of a lung area image of a general patient to which a plurality of intensity sections are applied according to another embodiment of the present invention; and
FIGS. 6 to 9 shows views each illustrating an example of an image of the lung area of a patient with pneumonia to which the plurality of intensity sections of FIG. 5 are applied according to an embodiment of the present invention.

### BEST MODES FOR IMPLEMENTATION OF THE DISCLOSURE

According to an aspect of the present invention, there is provided a medical image reading assistance apparatus for assisting the reading of chest medical images, the medical image reading assistance apparatus including a computing system, wherein the computing system includes at least one processor. In this case, the at least one processor is configured to: segment airways and blood vessels from a medical image including the lungs; segment small regions corresponding to a plurality of intensity sections divided and set based on at least one of the property and state of tissue in a lung area image generated by excluding the airways and the blood vessels from the medical image; and visualize the distributions of the small regions corresponding to the plurality of intensity sections within the lung area image.

The plurality of intensity sections may be set based on at least one of the clinically distinctive property and state of tissue.

The at least one processor may be further configured to threshold the small regions based on the plurality of intensity sections within the lung area image.

The at least one processor may be further configured to, within the lung area image, visualize the distribution of first small regions corresponding to a first intensity section on a first window and also visualize the distribution of second small regions corresponding to a second intensity section on a second window.

The at least one processor may be further configured to, within the lung area image, visualize the distribution of first small regions corresponding to a first intensity section and also visualize the distribution of second small regions corresponding to a second intensity section by overlaying the distribution of second small regions on the distribution of first small regions.

The at least one processor may be further configured to provide a user menu configured to allow a user to reset the plurality of intensity sections or add one or more intensity sections to the plurality of intensity sections.

According to another aspect of the present invention, there is provided a medical image reading assistance apparatus for assisting the reading of chest medical images, the medical image reading assistance apparatus including a computing system, wherein the computing system includes at least one processor. In this case, the at least one processor is configured to: segment airways and blood vessels from a medical image including the lungs; segment first regions corresponding to a first intensity section set to include at least one of blood and thrombi in a lung area image generated by excluding the airways and the blood vessels from the medical image; and visualize the distribution of the first regions within the lung area image.

The first intensity section may be set to correspond to blood.

The first intensity section may be set to correspond to thrombi.

The at least one processor may be further configured to: segment second regions corresponding to the second intensity section set to correspond to blood within the lung area image; and visualize the distribution of the first regions and the distribution of the second regions so that the first regions and the second regions are distinguished from each other within the lung area image.

The at least one processor may be further configured to: segment third regions corresponding to a third intensity section set to correspond to ground-glass opacity (GGO) within the lung area image; and visualize the distribution of the first regions and the distribution of the third regions so that the first regions and the third regions are distinguished from each other within the lung area image.

The at least one processor may be further configured to threshold the first regions based on the first intensity section within the lung area image.

The at least one processor may be further configured to: quantify the distribution of the first regions within the lung area image; and generate reading assistance information regarding whether a lung disease is present in the lung area image and a cause of the lung disease based on information about the quantification of the distribution of the first regions.

According to still another aspect of the present invention, there is provided a medical image reading assistance method, the medical image reading assistance method being performed by a medical image reading assistance apparatus for assisting the reading of chest medical images, the medical image reading assistance apparatus including a computing system that includes at least one processor, the medical image reading assistance method being performed by program instructions that are executed by the at least one processor.

The medical image reading assistance method includes: segmenting airways and blood vessels in a medical image including the lungs; generating a lung area image by excluding the airways and the blood vessels from the medical image; segmenting small regions corresponding to a plurality of intensity sections divided and set based on at least one of the property and state of tissue in the lung area image; and visualizing the distributions of the small regions corresponding to the plurality of intensity sections within the lung area image.

According to still another aspect of the present invention, there is provided a medical image reading assistance method, the medical image reading assistance method including: segmenting airways and blood vessels in a medical image including the lungs; generating a lung area image by excluding the airways and the blood vessels from the medical image; segmenting first regions corresponding to a first intensity section set to include at least one of blood and thrombi in the lung area image; and visualizing the distribution of the first regions within the lung area image.

### MODES FOR IMPLEMENTATION OF THE DISCLOSURE

Other objects and features of the present invention in addition to the above-described objects will be apparent from the following description of embodiments given with reference to the accompanying drawings.

Embodiments of the present invention will be described in detail below with reference to the accompanying drawings. In the following description, when it is determined that a detailed description of a related known component or function may unnecessarily make the gist of the present invention obscure, it will be omitted.

The spirit of the present invention should not be understood to be limited by the examples. The same reference numerals in each figure may indicate the same elements. Length, height, size, width, and so on, which is introduced in the embodiments and drawings of the present invention may be understood to be exaggerated for better understanding.

Deep learning/CNN-based artificial neural network technology, which has recently developed rapidly, is considered for the purpose of identifying a visual element that is difficult to identify with the human eye when it is applied to the imaging field. The fields of application of the above technology are expected to expand to various fields such as security, medical imaging, and non-destructive testing.

For example, in the field of medical imaging, there are cases where a tissue in question is not immediately diagnosed as a cancer tissue in a biopsy state but whether it is a cancer tissue is determined only after being monitored from a pathological point of view. Although it is difficult to confirm whether a corresponding cell is a cancer tissue in a medical image with the human eye, there is an expectation that the application of artificial neural network technology may acquire more accurate prediction results than observation with the human eye.

It is expected that this artificial neural network technology is applied and performs the analysis process of detecting a disease or lesion difficult to identify with the human eye in a medical image, segmenting a region of interest such as a specific tissue, and measuring the segmented region.

As to recent medical images such as CT or MRI images, a series of medical images is acquired through a single acquisition process, and the series of medical images is not limited to a single type of lesion but may also be used to detect various types of lesions. For example, for the lungs, a lung nodule as well as chronic obstructive pulmonary disease (COPD) may be diagnosed, emphysema may be diagnosed, and/or chronic bronchitis and/or an airway-related disease may also be diagnosed.

Diagnosis using a medical image refers to a process in which a medical professional identifies a disease or lesion that has occurred in a patient. In this case, prior to diagnosis using a medical image, a medical professional analyzes the medical image and detects a disease or lesion appearing in the medical image. A primary opinion on the detection of a disease or lesion on a medical image is referred to as "findings," and the process of deriving findings by analyzing a medical image is referred to as "reading."

Diagnosis using a medical image is made in such a manner that a medical professional analyzes the findings, derived through the process of reading the medical image, again. In this process, role can be frequently shared in such a manner that a radiologist reads a medical image and derives findings and a clinician derives a diagnosis based on a reading result and the findings.

The assistance of the diagnosis of a medical image by an artificial intelligence has a considerably comprehensive meaning, and may be classified into the case of assisting the medical diagnosis by providing findings based on a medical image processing and/or analysis result, the case of assisting the process of reading a medical image, the case of assisting the diagnosis of the reading result of a medical image, and the case of assisting decision-making on a medical action such as treatment, administration, or surgery based on the diagnosis result of a medical image. The artificial intelligence can assist at least a part of the process of medical diagnosis by analyzing/processing the medical images such as detecting lesion/illness, segmenting an ROI, classification, quantitative measurement, and/or decision-making. The artificial intelligence can assist at least a part of the process of medical diagnosis by providing and/or generating additive/incidental information requested by the medical professionals.

In the above-described related documents, lesion candidates are detected using an artificial neural network and classified, and findings are then generated. Each of the findings includes diagnosis assistance information, and the diagnosis assistance information may include quantitative measurements such as the probability that the finding corresponds to an actual lesion, the confidence of the finding, and the malignity, size and volume of the corresponding one of the lesion candidates to which the findings correspond.

In medical image reading assistance using an artificial neural network, each finding must include numerically quantified probability or confidence as diagnosis assistance information. Since all findings may not be provided to a user, the findings are filtered by applying a predetermined threshold, and only passed findings are provided to the user.

Some of the contents disclosed in these related art documents may be related to the objects to be achieved by the present invention, and some of the solutions adopted by the present invention may be applied to these related art documents in the same manner.

The present invention is directed to a system for assisting the reading of medical images that provides a configuration for visualizing various analysis technologies, to which an artificial neural network technology and a computer aided diagnosis (CAD) technology are applied, in the most appropriate form that can be read by human professionals.

Some or all of the configurations of Korean Patent Application Publication No. 10-2019-0090986 entitled "System and Method for Assisting Reading of Chest Medical Images," Korean Patent Application Publication No. 10-2020-0046507 entitled "Method of Providing Information for Diagnosis of Pulmonary Fibrosis and Computing Apparatus for Diagnosing Pulmonary Fibrosis," and Korean Patent No. 10-1587719 entitled "Medical Image Analysis Apparatus and Method for Distinguishing between Pulmonary Nodule and Pulmonary Vessel" may be applied to the present invention as some of the solutions adopted by the present invention within a range related to any one of the objects of the present invention.

In other words, some or all of the configurations of the conventional technologies may be included in the present invention in order to embody the present invention, and some or all of the configurations of the conventional technologies that are included in the present invention will be regarded as part of the present invention. Hereinafter, a technology for the diagnosis of lung disease, particularly pneumonia, which is the main application object of the present invention, and the necessity thereof will be disclosed within a scope consistent with any one of the objects of the present invention.

Pneumonia concerns an inflammatory condition in the lungs that primarily affects small air sacs known as alveoli. Symptoms of pneumonia typically include a combination of dry cough, chest pain, fever, and difficulty with breathing. The severity of symptoms is considerably variable.

Pneumonia is usually caused by infection with a virus or bacteria, and may be less commonly caused by another microorganism, a specific drug, or a condition such as autoimmune disease. Pneumonia may have risk factors such as cystic fibrosis, chronic obstructive pulmonary disease (COPD), sickle cell disease, asthma, diabetes, heart failure, a history of smoking, inability to cough (after stroke), and a weakened immune system. The diagnosis of pneumonia is often based on symptoms and physical examination, and the treatment of pneumonia depends on an underlying cause. For example, pneumonia caused by bacteria is treated with antibiotics. When pneumonia is severe, an infected person is usually hospitalized, and oxygen therapy may be applied when an oxygen level is low.

A chest CT image, a blood test, and a culture of sputum may help to confirm the diagnosis of pneumonia. New true direct measured, non-extrapolated 3D volumetric CT imaging biomarkers are more specific in the differentiation of causes of pneumonia, and may quantify other components of the disease. In addition, the volumes of various disease components (infiltration (as in the case of GGO), consolidation, fluids, interstitial congestion, transudate, exudate, blood, thrombi, embolus, and organizing and fibrosing tissue) may be quantified and expressed in milliliters or as percentages within the total lung volume. These biomarker values are indicative of the diagnosis, stage and prognosis of the disease. CT image biomarkers are applicable as thresholds for hospital admission, intensive care (IC), and active ventilation. In addition, such CT image biomarkers may be used to determine whether to discharge patients.

Each year, pneumonia affects about 450 million people (7% of the world population) worldwide and causes about 4 million deaths. Despite the dramatic improvement in survival rates attributable to the introduction of antibiotics and vaccines in the 20th century, pneumonia remains a leading cause of death in the developing world for people who are very old, very young, or have chronic illnesses.

As described above, the intensity of a CT image varies depending on the characteristics of human organs and tissues in a CT image. Accordingly, a technology for identifying a normal tissue and a lesion by segmenting regions having similar intensity values is obvious to those skilled in the art.

An embodiment of the present invention discloses technology configured to set various intensity sections based on the state of tissue inside a lung area and/or the nature of the tissue and visualize the intensity sections, thereby assisting medical staff in identifying symptoms of lung disease and then obtaining additional information about various factors that are causes of the symptoms.

A technology for setting intensity sections based on a specific tissue, a specific state of the specific tissue, and a property of the specific tissue that is proposed in the embodiments of the present invention is not a new item that differentiates the prevent invention from the conventional technologies. In contrast, a configuration for the update of clinical information attributable to clinical research, the optimization of a diagnostic imaging apparatus (modality), and/or the optimization or update of a threshold value for each intensity section attributable to the analysis of medical images is newly proposed in the present invention.

An embodiment of the present invention newly proposes a technology for setting a plurality of intensity sections based on a specific tissue, a specific state of tissue, and/or a specific property of tissue and visualizing the plurality of intensity sections to facilitate comparison therebetween. This feature of the present invention is a new technology differentiated from the conventional technologies.

The setting of a plurality of intensity sections proposed in an embodiment of the present invention and a combination of a plurality of intensity sections for visualization may be updated and optimized in response to the update of clinical information attributable to clinical research.

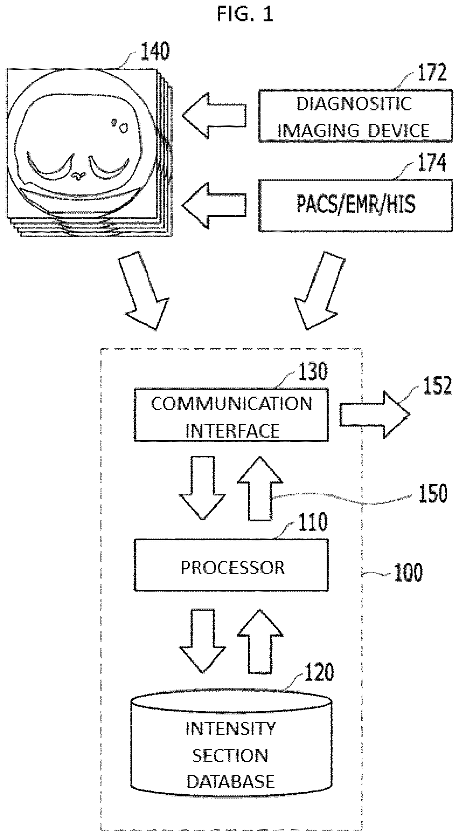

FIG. 1 is a drawing showing a medical image reading assistance apparatus for assisting the reading of chest medical images according to an embodiment of the present invention.

Referring to FIG. 1, the medical image reading assistance apparatus according to the present embodiment includes a computing system 100, and the computing system 100 includes at least one processor 110. The computing system 100 may further include an intensity section database 120. The at least one processor 110 may receive a medical image 140 via a communication interface 130, may perform image processing on the medical image 140 in cooperation with the intensity section database 120, and may generate reading assistance information 150 as a result of the image processing performed on the medical image 140.

The medical image 140 may be acquired by a diagnostic imaging apparatus (modality) 172, or may be transmitted from a database 174 outside the computing system 100. The external database 174 may be implemented by including at least one of a hospital medical image, a hospital medical record, and an information database such as a picture archiving and communication system (PACS), an electronic medical record (EMR), and a hospital information system (HIS).

The communication interface 130 may transmit visualization information 152, to be provided to a user by a user interface (not shown) based on the reading assistance information 150, to the user interface. The user interface may include a display, and may further include a known input means through which a user can input a command, such as a touch screen, a keyboard, a keypad, and/or a mouse.

The reading assistance information 150 may include information obtained by visualizing the distributions of small regions corresponding to intensity sections, which are the core spirit of the present invention to be described later. The reading assistance information 150 may include information generated to target a general user interface, and the communication interface 130 may generate the visualization information 152 in which the reading assistance information 150 is optimized for the user interface based on the specifications, screen size, resolution, and color provision capability of the user interface.

It will be apparent to those skilled in the art that although an example in which the visualization information 152 and the reading assistance information 150 are separate from each other is disclosed in the embodiment of FIG. 1, the visualization information 152 and the reading assistance information 150 may match each other and also the at least one processor 110 may generate the reading assistance information 150 and the visualization information 152 optimized for the user interface by taking into consideration the specifications of the user interface in another embodiment of the present invention.

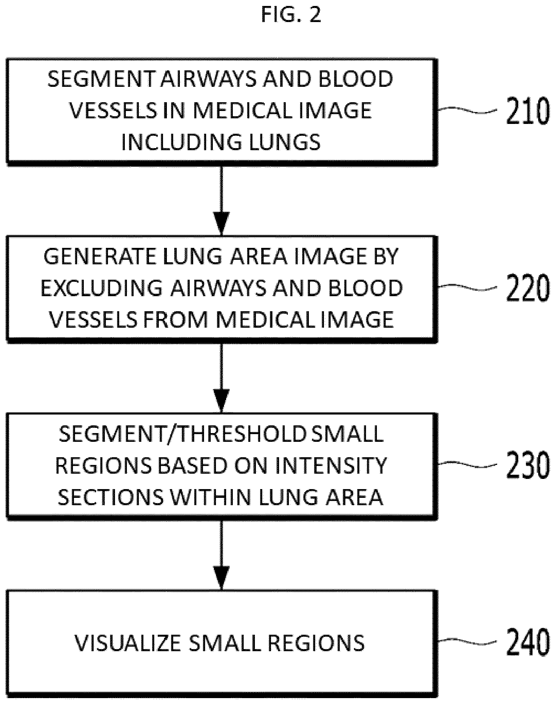

FIG. 2 is a diagram showing the sub-modules of a program instruction set executed in a medical image reading assistance apparatus according to an embodiment of the present invention and also showing the main process of a medical image reading assistance method performed according to the execution of the sub-modules. The sub-modules 210, 220, 230, and 240 of the program instruction set perform given functions. The logical sequence of the operations of the sub-modules 210, 220, 230, and 240 may be understood as the logical sequence of the operations of a medical image reading assistance method according to an embodiment of the present invention. The logical sequence of the operations of the submodules 210, 220, 230, and 240 of FIG. 2 may be understood as the operational flow of the medical image reading assistance method.

Referring to FIGS. 1 and 2 together, the at least one processor 110 segments airways and blood vessels in a medical image 140 including the lungs (see segmentation 210).

The at least one processor 110 generates a lung area image by excluding the airways and the blood vessels from the medical image 140 (see 220). In this case, the blood vessels that are segmented and excluded through the image processing of the medical image 140 are blood vessels having a considerable size that can be identified even with the naked eye. Micro-blood vessels that exchange oxygen in the lung area do not appear during the process of the segmentation 210, and may be included in the lung area image without being excluded from the lung area image. The distributions of blood and/or thrombus values in these micro-blood vessels may be used as reference information for identifying, diagnosing, and making decisions for pneumonia caused by COVID-19, as described below.

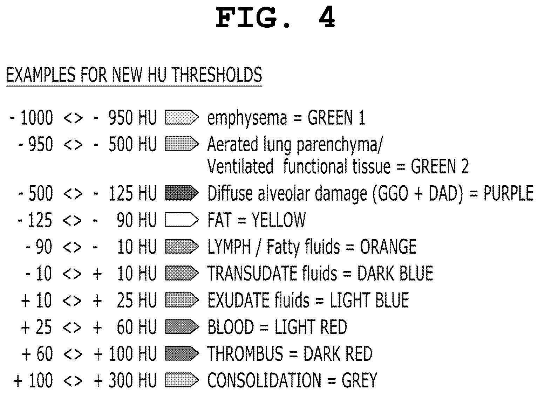

The at least one processor 110 segments small regions corresponding to intensity sections in the lung area image (segmentation 230). For information about the plurality of intensity sections, reference may be made to the intensity section database 120. The plurality of intensity sections may be divided and set based on at least one of the properties and states of a tissue. Examples of the information about the plurality of intensity sections will be specifically disclosed in the embodiments of FIGS. 4 to 9 to be described later.

The at least one processor 110 may visualize the distributions of small regions corresponding to the plurality of intensity sections in the lung area image (see 240). In this case, the distributions of the small regions may be visualized by being overlaid on the medical image 140. Information about the visualization of the distributions of the small regions may be generated as the reading assistance information 150 by at least one processor 110.

The at least one processor 110 may threshold the small regions based on the plurality of respective intensity sections within the lung area image. In other words, the segmentation of the small regions may specifically be thresholding based on the intensity sections.

The plurality of intensity sections may be set based on at least one of the clinically classified properties or states of a tissue. For example, when subdividing properties of a tissue to diagnose a specific disease and/or the cause of the disease has a clinical significance, the tissue that was previously subjected to thresholding as a single section may be subjected to thresholding as subdivided sections. For example, a blood/thrombus intensity section, which was conventionally subjected to thresholding as a single section, may be divided into and subjected to thresholding as a blood intensity section and a thrombus intensity section to identify pneumonia symptoms caused by COVID-19. Alternatively, when the distribution of fat tissues needs to be visualized together with the distribution of ground-glass opacity (GGO) in order to identify other causes of pneumonia, fat tissue intensity sections and GGO intensity sections may be set, and fat tissue regions and GGO regions may be subjected to thresholding.

In an embodiment of the present invention, the at least one processor 110 may visualize the distribution of first small regions, corresponding to a first intensity section in the lung area image, on a first window, and the distribution of second small regions corresponding to a second intensity section may be visualized on a second window. In other words, different intensity sections may be visualized on respective images. In this case, the individual images may be expressed such that a user/medical staff can easily compare the distribution of the first intensity section and the distribution of the second intensity section by providing the same view. In another embodiment, the individual images may be visualized such that a user/medical staff can intuitively compare the distribution of the first intensity section and the distribution of the second intensity section by providing different views.

In another embodiment of the present invention, the at least one processor 110 may visualize the distribution of first small regions corresponding to a first intensity section in the lung area image, and the distribution of second small regions corresponding to a second intensity section may be visualized by being overlaid on the distribution of first small regions. In this case, the distribution of first small regions and the distribution of second small regions may be visualized by being overlaid on one view of the medical image 140. In this case, the priorities based on which the intensity sections and the small regions are to be displayed may be pre-designated and stored in the intensity section database 120. The priorities may be pre-designated based on clinical significance and/or user preference. Each of the priorities may refer to the priority based on which an object is displayed above such that it can be easily identified with the naked eye.

Referring back to FIG. 1, the at least one processor 110 may provide a user menu configured to allow a user to reset or add one or more sections to a plurality of intensity sections. For example, a user menu configured to allow a user to reset or add one or more sections to a plurality of intensity sections based on clinical importance when clinical knowledge is updated may be provided. For example, it may be assumed that existing clinical knowledge is updated with the clinical knowledge in which the distribution of thrombi increases in the lung area or throughout the human body as one of the symptoms of pneumonia caused by COVID-19.

In a medical image reading assistance apparatus according to an embodiment of the present invention, at least one processor 110 and an intensity section database 120 are combined to provide assistance so that respective regions can be divided according to the type, property, and/or state of tissue having a clinical significance. For information that assists a medical staff in the reading of medical images, diagnosis, and decision making, reference may be made to the individual divided regions. The intensity section information stored in the intensity section database 120 is a means for additionally providing clinically significant information about one or more causes of a specific disease as well as one or more symptoms of the specific disease. The intensity section database 120 and the at least one processor 110 may function as a platform used to diagnose symptoms and causes for each disease.

When the clinical knowledge is updated, the settings of the intensity sections in the intensity section database 120 of the platform may be updated. It may be updated by a user or by the administrator of the intensity section database 120. In addition, even when the plurality of intensity sections are neither reset nor are one or more sections added to the plurality of intensity sections, information about the existing priorities of the intensity sections may be updated, and information about the relationship between the existing intensity sections and a specific disease may be updated.

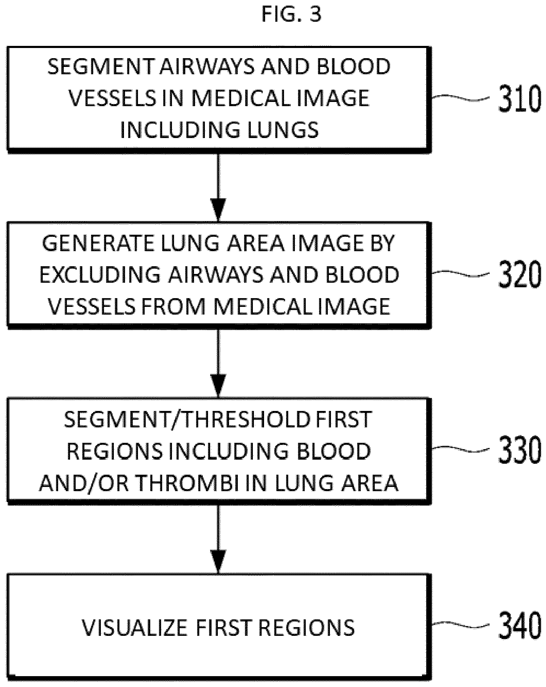

FIG. 3 is a diagram showing the sub-modules of a program instruction set executed in a medical image reading assistance apparatus according to an embodiment of the present invention and also showing the main process of a medical image reading assistance method performed according to the execution of the sub-modules. The sub-modules 310, 320, 330, and 340 of the program instruction set perform given functions. The logical sequence of the operations of the sub-modules 310, 320, 330, and 340 may be understood as the logical sequence of the operations of the medical image reading assistance method according to the present embodiment. The logical sequence of the operations of the sub-modules 310, 320, 330, and 340 of FIG. 3 may be understood as the operational flow of the medical image reading assistance method.

Referring to FIGS. 1 and 3 together, the at least one processor 110 segments airways and blood vessels in a medical image 140 including the lungs (see segmentation 310).

The at least one processor 110 generates a lung area image by excluding the airways and the blood vessels from the medical image 140 (see 320).

The at least one processor 110 segments first regions, including blood vessels and/or thrombi, in the lung area image (see segmentation 330). For information about the intensity sections for the blood vessels and/or thrombi, reference may be made to the intensity section database 120.

The at least one processor 110 may visualize the distribution of the first regions in the lung area image (see 340). In this case, the distribution of the first regions may be visualized by being overlaid on the medical image 140. Information about the visualization of the distribution of the first regions may be generated as the reading assistance information 150 by the at least one processor 110.

The at least one processor 110 may threshold the first regions based on first intensity sections within the lung area image. In other words, the segmentation of the first regions may specifically be thresholding based on the first intensity sections.

In an embodiment, the first intensity sections may be set to correspond to blood.

In another embodiment, the first intensity sections may be set to correspond to thrombi.

When the first intensity sections correspond to thrombi, the at least one processor 110 may segment second regions, corresponding to second intensity sections set to correspond to blood, in the lung area image (segmentation), and may visualize the distribution of the first regions and the distribution of the second regions so that the first regions and the second regions can be distinguished in the lung area image. In this case, the reading assistance information 150 may include information about the visualization of the distribution of the first regions and the distribution of the second regions.

The at least one processor 110 may segment third regions, corresponding to third intensity sections set to correspond to ground-glass opacity (GGO) values, in the lung area image, and may visualize the distribution of the first areas and the distribution of the third areas so that the first areas and the third areas can be distinguished from each other. In this case, the reading assistance information 150 may include information about the visualization of the distribution of the first regions and the distribution of the third regions.

Referring back to FIG. 1, the at least one processor 110 may quantify the distribution of the first regions in the lung area image, and may generate reading assistance information 150 regarding whether a lung disease is present in the lung area image and the cause of the lung disease based on information about the quantification of the distribution of the first regions. In other words, the reading assistance information 150 may include not only the information about the quantification of the distribution of the first regions but also a report based on the quantification and/or statistics of the distribution of the first regions. This report may assist medical staff in diagnosis and decision making. For example, for the possibility that a symptom appearing in the medical image 140 corresponds to pneumonia caused by COVID-19, quantification and/or statistical information to which medical staff may refer may be provided together with the visualization information 152 as a report. The distribution of GGO may be understood as an index indicating a symptom of pneumonia, and the distribution of thrombi may be understood as an index indicating the possibility that the symptom corresponds to pneumonia caused by COVID-19. In other words, when the distribution of GGO and the distribution of thrombi are visualized and statistically processed together, this information may be used as information for the diagnosis of pneumonia caused by COVID-19.

FIG. 4 is a diagram illustrating an embodiment of a plurality of intensity sections based on a plurality of properties and states of clinical tissues applied in a medical image reading assistance apparatus according to an embodiment of the present invention. The setting of the plurality of intensity sections shown in FIG. 4 is an embodiment of the present invention, and may be optimized or updated based on the update of clinical information attributable to clinical research, the optimization of a diagnostic imaging apparatus (modality), and/or the optimization of a threshold value attributable to the analysis of medical images.

Emphysema and aerated lung parenchyma (a ventilated functional tissue) may designated as separate intensity sections, and may be divided as separate regions and then visualized. The distribution of emphysema may be used as an index indicating a symptom of chronic obstructive pulmonary disease (COPD). Aerated lung parenchyma (a ventilated functional tissue) is generally regarded as a tissue that normally performs a lung function.

GGO refers to local nodular pulmonary infiltration, and is generally defined as a nodule in which the boundaries of bronchi or blood vessels are drawn desirably. Diffuse alveolar damage (DAD) and GGO are generally treated as opacity (OP), and the distribution of opacity (OP) is regarded as an index indicating a symptom of pneumonia.

Intensity sections may be set for fat tissues, lymphatic tissues/lymphedema, transudate fluids, and exudate fluids, and small regions corresponding to the intensity sections may be subjected to thresholding and then visualized.

Furthermore, an intensity section may be set for a lung consolidation region in addition to pneumonia and COPD, and a lung consolidation region may be separately subjected to thresholding. The lung consolidation generally refers to a state in which a lung is hardened because the air in the alveoli is replaced with liquid or cells. Lung opacity relatively uniformly increases in an X-ray or CT image, and there is little change in lung volume, which can be seen on an air bronchogram. The lung consolidation may also refer to a case where opacity increases in a CT image to the extent that pulmonary vessels within a lesion cannot be distinguished.

As described above, symptoms of lung disease appear different depending on the type of original tissue, but different patterns appear even when the state of the tissue changes. In order to have a clinical significance, separate intensity sections may be set based on the type of tissue, the nature of the tissue, and/or the state of the tissue, and small regions corresponding to the intensity sections may be subjected to thresholding.

According to an embodiment of the present invention, various factors inside the lung area may be detected and provided to medical staff together with information about symptoms of lung disease. In an embodiment of the present invention, various intensity sections are set and visualized based on the states and/or properties of tissues inside the lung area, thereby assisting the medical staff in checking the symptoms of the lung disease and obtaining additional information about various factors that cause the lung disease.

For example, a lung disease caused by COVID-19 may have a different pattern from that of a common bacterial lung disease. In the embodiment of the present invention, additional information about this pattern may be identified inside the lung area, visualized, and provided to medical staff together with information about symptoms of the lung disease. The medical staff reads a chest medical image while taking into consideration various factors for the causes of the symptoms of the lung disease, and an embodiment of the present invention assists the medical staff in reading the chest medical image.

Due to the recent epidemic of COVID-19, it has been recognized by medical staff that if the causes of lung diseases are different even when the symptoms of the lung diseases are similar, treatment methods and the types of drugs to be administered need to be completely different. However, since the conventional medical image reading assistance technology provides only information about symptoms of lung disease, it has been difficult to assist medical staff in diagnosis and decision making. In an embodiment of the present invention, information about symptoms of lung disease and additional information for the estimation of the causes of the symptoms may be provided together, thereby assisting medical staff in reading, diagnosis, and decision making.

In an embodiment of the present invention, additional information for distinguishing between a lung disease caused by COVID-19 and a general bacterial lung disease may be visualized, thereby assisting medical staff in rapid and accurate decision making.

In an embodiment of the present invention, there may be implemented a platform that provides a user menu to allow a user to reset or add one or more intensity sections of clinical tissue based on updated clinical knowledge when the clinical knowledge on a cause of lung disease is updated and also provides the user with reading assistance information about various causes of lung disease together with information about symptoms of the lung disease.

In an embodiment of the present invention, the distribution of specific intensity regions for a specific cause of lung disease may be quantified, reading assistance information about whether the lung disease is present and the specific cause of the lung disease may be generated based on the distribution of the specific intensity regions, and then the reading assistance information may be provided to a user.

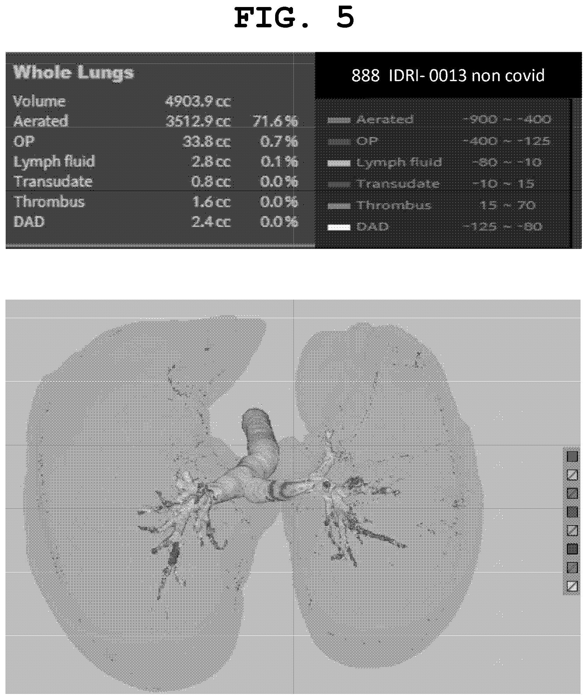

FIG. 5 shows views illustrating an example of a lung area image of a general patient to which a plurality of intensity sections are applied according to another embodiment of the present invention. In FIG. 5, there is disclosed an example in which the setting of intensity sections different from that of FIG. 4 is applied. As described above, the setting of intensity sections may be optimized or updated based on the update of clinical information attributable to clinical research, the optimization of a diagnostic imaging apparatus (modality), and/or the optimization of a threshold value attributable to the analysis of medical images.

Referring to FIGS. 1 and 5 together, the at least one processor 110 may generate the reading assistance information 150 so that information about the intensity sections can be displayed in connection with the distribution of small regions when information related to the visualization of the distribution of small regions is generated. In this case, the information about intensity sections displayed together in connection with the distribution of small regions may be displayed to include the ranges of intensity values and the clinical significances/names of the information about intensity sections.

Furthermore, quantified measured values and/or statistics may be displayed for the small regions corresponding to the intensity sections. The measured values and/or the statistics may include the areas of the small regions and/or the ratios of the areas of the small regions to the total lung area.

In FIG. 5, it can be seen that an image of the lung area of a subject in a relatively desired condition is shown, the percentage of an opacity (OP) area is 0.7%, and the percentage of thrombi is less than 0.1%. The subject of FIG. 5 has relatively mild pneumonia, which is not pneumonia caused by COVID-19.

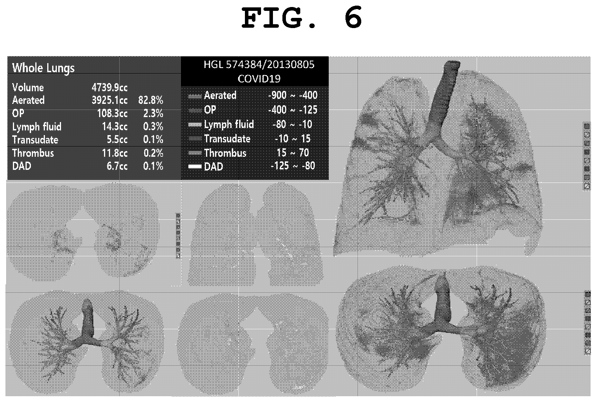

FIG. 6 shows views illustrating an example of an image of the lung area of a patient with pneumonia to which the plurality of intensity sections(intensity ranges clinical meanings/names/significances assigned thereto) of FIG. 5 are applied according to an embodiment of the present invention.

In FIG. 6, there is shown an embodiment in which intensity section regions(regions segmented/thresholded in image corresponding to intensity section) are visualized in respective windows. In addition, there are presented intensity (value) ranges(with upper/lower bound of concrete values of CT intensity) for the respective intensity sections, the clinical meanings/names/significances of the respective intensity sections(or intensity section regions), and quantified measured values/statistics for the respective intensity section regions.

It can be seen that the subject of FIG. 6 suffers from more severe pneumonia symptoms than the subject of FIG. 5. In FIG. 6, the occupancy of opacity OP regions is 2.3%. In addition, it can be seen that since the occupancy of thrombus regions is 0.2%, the possibility that the occupancy of thrombus regions exceeds 10% of the opacity (OP) regions and pneumonia in question is pneumonia caused by COVID-19 is higher than that for the subject of FIG. 5.

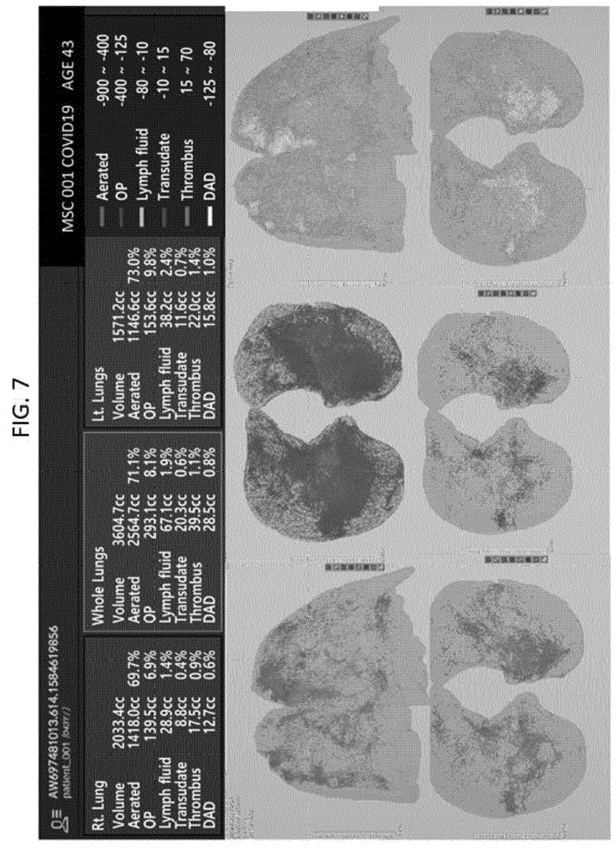

FIG. 7 shows views illustrating an example of an image of the lung area of a patient with pneumonia to which the plurality of intensity sections of FIG. 5 are applied according to an embodiment of the present invention.

Referring to FIG. 7, there is shown an embodiment of the visualization of an image of the lung area of a patient with pneumonia caused by COVID-19. In FIG. 7, regions of intensity sections are visualized in respective windows, and separate quantified measured values/statistics for the whole lungs, the left lung, and the right lung are presented for each of the intensity section regions. The pneumonia patient of FIG. 7 suffers from more severe pneumonia than the subjects of FIGS. 5 and 6, and this pneumonia is much more likely to be the pneumonia caused by COVID-19. In addition, it can be seen that the left lung has the higher occupancies of the opacity regions and the thrombus regions than the right lung and is subjected to the more significant influences of pneumonia symptoms and COVID-19.

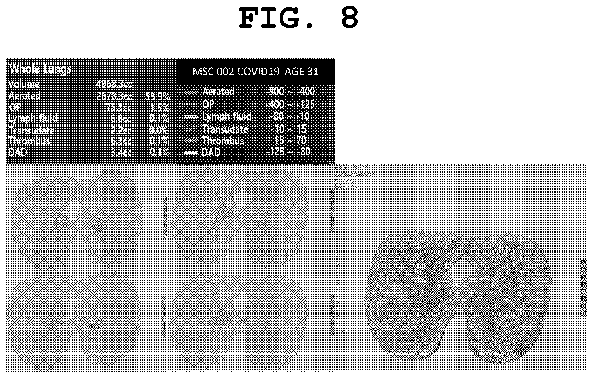

FIG. 8 shows views illustrating an example of an image of the lung area of a patient with pneumonia to which the plurality of intensity sections of FIG. 5 are applied according to an embodiment of the present invention.

Referring to FIG. 8, there is shown an embodiment of the visualization of an image of the lung area of a patient with pneumonia attributable to COVID-19. In FIG. 8, there is illustrated an embodiment in which the regions of a plurality of intensity sections are visualized by being overlaid on one window. The combination of the regions of intensity sections overlaid on one window may be pre-designated according to the type of diagnosis target disease, or may be selected by the user. In FIG. 8, intensity ranges(with upper/lower bound of concrete values of CT intensity), clinical names/descriptions, and separate quantified measured values/statistics are presented for the individual intensity section regions.

It can be seen that the subject of FIG. 8 has relatively mild pneumonia symptoms, but this pneumonia is highly likely to be pneumonia caused by COVID-19.

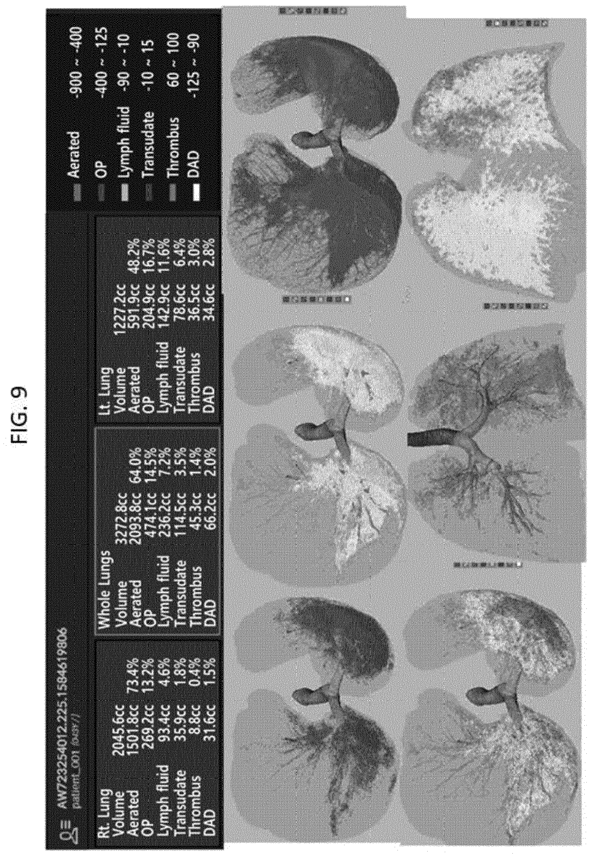

FIG. 9 shows views illustrating an example of an image of the lung area of a patient with pneumonia to which the plurality of intensity sections of FIG. 5 are applied according to an embodiment of the present invention.

Referring to FIG. 9, there is shown an embodiment of the visualization of an image of the lung area of a patient with pneumonia caused by COVID-19. In FIG. 9, there is shown an embodiment in which the regions of a plurality of intensity sections are overlaid on one window and visualized. The combination of the regions of intensity sections overlaid on one window may be pre-designated according to the type of diagnosis target disease, or may be selected by a user. In FIG. 9, intensity ranges, clinical names/descriptions, and separate quantified measured values/statistics are also presented for the individual intensity section regions.

When the regions of a plurality of intensity sections are overlaid on one window, a priority is determined for each of the regions of a plurality of intensity sections such that a layer to be displayed above is determined. In addition, separate quantified measured values/statistics for the whole lungs, the left lung, and the right lung are presented for each of the regions of a plurality of intensity section regions.

It can be seen that the subject of FIG. 9 also has more severe pneumonia symptoms and this pneumonia is much more likely to be pneumonia caused by COVID-19.

The regions of intensity sections overlaid on one window may be preset by taking into consideration a clinical significance. In this case, regions having a high degree of overlapping with each other may be displayed on one window, or regions having a low degree of overlapping with each other may be displayed on one window. In addition, the regions of intensity sections overlaid on one window are visualized based on predetermined priorities. The combination of the regions of intensity sections overlaid on one window and information about the priorities of the regions of the intensity sections may be updated or optimized based on various causes described above.

According to the present invention, there may be implemented technology for assisting the reading of chest medical images that can not only detect a symptom of lung disease, but also provide additional information about a cause of the lung disease.

According to the present invention, various factors inside a lung area may be detected and medical staff may be provided with information about the factors together with information about a symptom of lung disease. Various intensity sections may be set based on the state of tissue inside a lung area and/or the nature of the tissue and then visualized, thereby assisting medical staff in identifying a symptom of lung disease and then obtaining additional information about various factors.

According to the present invention, additional information for distinguishing between a lung disease caused by COVID-19 and a general bacterial lung disease may be visualized, thereby assisting medical staff in rapid and accurate decision making.

According to the present invention, a user menu may be provided to allow a user to reset or add one or more intensity sections of clinical tissue based on updated clinical knowledge when the clinical knowledge on a cause of lung disease is updated. There may be implemented a platform that provides a user with reading assistance information about various causes of lung disease together with information about symptoms of the lung disease.

According to the present invention, the distribution of specific intensity regions for a specific cause of lung disease may be quantified. Furthermore, reading assistance information about whether the lung disease is present and the specific cause of the lung disease may be generated based on the distribution of the specific intensity regions and then provided to a user.

The method according to an embodiment of the present disclosure may be implemented as a computer-readable program or code on computer-readable recording media. Computer-readable recording media include all types of recording devices in which data readable by a computer system are stored. The computer-readable recording media may also be distributed in a network-connected computer system to store and execute computer-readable programs or codes in a distributed manner.

The computer-readable recording medium may also include a hardware device specially configured to store and execute program instructions, such as a read only memory (ROM), a random access memory (RAM), and a flash memory. The program instructions may include not only machine language codes such as those generated by a compiler, but also high-level language codes that executable by a computer using an interpreter or the like.

Although some aspects of the present disclosure have been described in the context of an apparatus, it may also represent a description according to a corresponding method, wherein a block or apparatus corresponds to a method step or feature of a method step. Similarly, aspects described in the context of a method may also represent a corresponding block or item or a corresponding device feature. Some or all of the method steps may be performed by (or using) a hardware device, e.g., a microprocessor, a programmable computer, or an electronic circuit. In some embodiments, one or more of the most important method steps may be performed by such an apparatus.

In embodiments, a programmable logic device, e.g., a field programmable gate array, may be used to perform some or all of the functions of the methods described herein. In embodiments, the field programmable gate array may operate in conjunction with a microprocessor to perform one of the methods described herein. In general, the methods are preferably performed by a certain hardware device.

Although described above with reference to the preferred embodiments of the present disclosure, it should be understood that those skilled in the art can variously modify and change the present disclosure within the scope without departing from the spirit and scope of the present disclosure as set forth in the claims below.

### INDUSTRIAL APPLICABILITY

Disclosed therein are a medical image reading assistance apparatus and method for assisting the reading of chest medical images. The medical image reading assistance apparatus includes a computing system, and the computing system includes at least one processor. The at least one processor segments airways and blood vessels from a medical image including the lungs, segments small regions corresponding to a plurality of intensity sections divided and set based on at least one of the property and state of tissue in a lung area image generated by excluding the airways and the blood vessels from the medical image, and visualizes the distributions of the small regions corresponding to the plurality of intensity sections within the lung area image.

## Claims

1. A medical image reading assistance apparatus for assisting reading of chest medical images, the medical image reading assistance apparatus comprising a computing system, wherein the computing system comprises at least one processor, and wherein the at least one processor is configured to:
segment airways and blood vessels from a medical image including lungs;
segment small regions corresponding to a plurality of intensity sections divided and set based on at least one of a property and state of tissue in a lung area image generated by excluding the airways and the blood vessels from the medical image; and
visualize distributions of the small regions corresponding to the plurality of intensity sections within the lung area image.

2. The medical image reading assistance apparatus of claim 1, wherein the plurality of intensity sections are set based on at least one of a clinically distinctive property and state of tissue.

3. The medical image reading assistance apparatus of claim 1, wherein the at least one processor is further configured to threshold the small regions based on the plurality of intensity sections within the lung area image.

4. The medical image reading assistance apparatus of claim 1, wherein the at least one processor is further configured to, within the lung area image, visualize a distribution of first small regions corresponding to a first intensity section on a first window and also visualize a distribution of second small regions corresponding to a second intensity section on a second window.

5. The medical image reading assistance apparatus of claim 1, wherein the at least one processor is further configured to, within the lung area image, visualize a distribution of first small regions corresponding to a first intensity section and also visualize a distribution of second small regions corresponding to a second intensity section by overlaying the distribution of second small regions on the distribution of first small regions.

6. The medical image reading assistance apparatus of claim 1, wherein the at least one processor is further configured to provide a user menu configured to allow a user to reset the plurality of intensity sections or add one or more intensity sections to the plurality of intensity sections.

7. A medical image reading assistance apparatus for assisting reading of chest medical images, the medical image reading assistance apparatus comprising a computing system, wherein the computing system comprises at least one processor, and wherein the at least one processor is configured to:
segment airways and blood vessels from a medical image including lungs;
segment first regions corresponding to a first intensity section set to include at least one of blood and thrombi in a lung area image generated by excluding the airways and the blood vessels from the medical image; and
visualize a distribution of the first regions within the lung area image.

8. The medical image reading assistance apparatus of claim 7, wherein the first intensity section is set to correspond to blood.

9. The medical image reading assistance apparatus of claim 7, wherein the first intensity section is set to correspond to thrombi.

10. The medical image reading assistance apparatus of claim 9, wherein the at least one processor is further configured to:
segment second regions corresponding to the second intensity section set to correspond to blood within the lung area image; and
visualize a distribution of the first regions and a distribution of the second regions so that the first regions and the second regions are distinguished from each other within the lung area image.

11. The medical image reading assistance apparatus of claim 7, wherein the at least one processor is further configured to:
segment third regions corresponding to a third intensity section set to correspond to ground-glass opacity (GGO) within the lung area image; and
visualize a distribution of the first regions and a distribution of the third regions so that the first regions and the third regions are distinguished from each other within the lung area image.

12. The medical image reading assistance apparatus of claim 7, wherein the at least one processor is further configured to threshold the first regions based on the first intensity section within the lung area image.

13. The medical image reading assistance apparatus of claim 7, wherein the at least one processor is further configured to:
quantify a distribution of the first regions within the lung area image; and
generate reading assistance information regarding whether a lung disease is present in the lung area image and a cause of the lung disease based on information about the quantification of the distribution of the first regions.

14. A medical image reading assistance method, the medical image reading assistance method being performed by a medical image reading assistance apparatus for assisting reading of chest medical images, the medical image reading assistance apparatus including a computing system that includes at least one processor, the medical image reading assistance method being performed by program instructions that are executed by the at least one processor, the medical image reading assistance method comprising:
segmenting airways and blood vessels in a medical image including lungs;
generating a lung area image by excluding the airways and the blood vessels from the medical image;
segmenting small regions corresponding to a plurality of intensity sections divided and set based on at least one of a property and state of tissue in the lung area image; and
visualizing distributions of the small regions corresponding to the plurality of intensity sections within the lung area image.

15. The medical image reading assistance method of claim 14, wherein the segmenting small regions comprises performing thresholding on the small regions based on the plurality of intensity sections within the lung area image.

16. A medical image reading assistance method, the medical image reading assistance method being performed by a medical image reading assistance apparatus for assisting reading of chest medical images, the medical image reading assistance apparatus including a computing system that includes at least one processor, the medical image reading assistance method being performed by program instructions that are executed by the at least one processor, the medical image reading assistance method comprising:
segmenting airways and blood vessels in a medical image including lungs;
generating a lung area image by excluding the airways and the blood vessels from the medical image;
segmenting first regions corresponding to a first intensity section set to include at least one of blood and thrombi in the lung area image; and
visualizing a distribution of the first regions within the lung area image.

17. The medical image reading assistance method of claim 16, further comprising:
segmenting third regions corresponding to a third intensity section set to correspond to ground-glass opacity (GGO) within the lung area image; and
visualizing a distribution of the first regions and a distribution of the third regions so that the first regions and the third regions are distinguished from each other within the lung area image.

18. The medical image reading assistance method of claim 16, further comprising:
quantifying a distribution of the first regions within the lung area image; and
generating reading assistance information regarding whether a lung disease is present in the lung area image and a cause of the lung disease based on information about the quantification of the distribution of the first regions.
